# FASCICULE DE BREVET EUROPEEN

(11) **EP 1 037 741 B1**
(45) Date de publication et mention de la délivrance du brevet: **01.09.2004**
(21) Numéro de dépôt: 99947547.8
(22) Date de dépôt: 12.10.1999
(51) Int. Cl.: B32B 7/04, A61B 19/02

(54) **MATERIAU D'EMBALLAGE DE STERILISATION SCELLABLE**
VERSIEGEL- UND STERILISIERBARES VERPACKUNGSMATERIAL
SEALABLE STERILISING PACKAGING MATERIAL

(30) Priorité: 12.10.1998 FR 9812753
(43) Date de publication de la demande: 27.09.2000
(73) Titulaire: ARJO WIGGINS, 92130 Issy Les Moulineaux (FR)
(72) Inventeur: HERVE, Philippe, 66400 Reynes (FR); PARIS-JOLLY, Agnès, 38340 Voreppe (FR)
(86) Numéro de dépôt international: PCT/FR1999/002455
(87) Numéro de publication internationale: WO 2000/021745

(56) Documents cités:
- WO-A-96/16562
- US-A- 4 724 961
- US-A- 5 730 530
- DATABASE WPI Section Ch, Week 9005 Derwent Publications Ltd., London, GB; Class A18, AN 90-035009 XP002112231 & JP 01 314773 A (ASAHI CHEM IND CO LTD), 19 décembre 1989 (1989-12-19)

## Description

La présente invention concerne un matériau d'emballage de stérilisation scellable pour les dispositifs médicaux devant être stérilisés, ainsi que l'emballage de stérilisation lui-même.

On connaît déjà des emballages de stérilisation scellables pour des dispositifs médicaux devant être stérilisés, notamment d'instruments ou de matériels ré-utilisables, comme par exemples les sondes, scalpels, pinces, ciseaux, aiguilles.

En effet, pour stériliser les dispositifs médicaux, on peut utiliser un emballage de stérilisation scellable à chaud et/ou sous pression qui peut être un emballage souple ou semi-rigide sous forme d'un sachet, d'une pochette, d'une gaine ou d'un blister ou encore un emballage rigide.

L'emballage rigide est un container constitué d'un récipient en général en plastique et thermoformé qui contiendra les dispositifs médicaux à stériliser puis qui sera fermé par un opercule qui peut être une feuille de papier barrière aux micro-organismes et scellable. Cette feuille est une feuille barrière semblable à celle utilisée pour l'autre type d'emballage décrit ci-après.

L'emballage souple ou semi-rigide sous forme d'un sachet, d'une pochette, d'une gaine ou d'un blister est un emballage constitué d'une partie (1) qui peut être en matière synthétique et d'une feuille de papier (2) ayant une perméabilité spécifique, scellées entre elles sur un certain périmètre selon la forme voulue pour l'emballage, une ouverture plus ou moins grande étant laissée afin de pouvoir introduire les objets. Les objets à stériliser sont placés à l'intérieur de l'emballage puis on scelle complètement ledit emballage. La partie (1) en matière synthétique peut être un film thermoplastique comme le polyéthylène ou le polypropylène. Ce film est en général imperméable aux gaz et à la vapeur d'eau et de plus transparent afin de voir le contenu de l'emballage. A la place du film plastique, on peut utiliser aussi une feuille semblable à la feuille de papier (2) qui a une perméabilité spécifique ou une feuille de papier enduite d'un produit scellant comme une couche de polyéthylène extrudé ou du poly (acétate de vinyle).
Dans le cas d'un blister on utilise un film plastique souple qui est thermoformé selon la forme du dispositif à emballer.

La feuille de papier (2) a une perméabilité spécifique qui la rend barrière aux micro-organismes mais qui permet de laisser passer les agents stérilisants afin de réaliser la stérilisation de l'emballage fermé et de son contenu par des méthodes de stérilisation utilisants comme agents stérilisants la vapeur d'eau ou des gaz stérilisants comme l'oxyde d'éthylène. L'emballage peut aussi être stérilisé par des rayonnements ionisants comme les rayonnements gamma ou béta.
Ces emballages offrent avantageusement la possibilité d'emballer individuellement les objets et de n'être ouverts si nécessaire qu'au moment de l'utilisation de l'objet stérilisé. Ils permettent donc un stockage des objets stérilisés dans de bonnes conditions de stérilité.

De façon plus détaillée, la feuille de papier (2) utilisable pour former ces emballages est obtenue par voie humide selon un procédé papetier par égouttage d'une suspension aqueuse de fibres de cellulose comportant en général un agent de résistance humide. De plus un agent de cohésion peut être introduit soit en masse dans la suspension aqueuse des fibres, soit par un traitement en surface de la feuille humide, pour renforcer mécaniquement la feuille. Par ailleurs un agent de collage peut être introduit aussi soit en masse dans la suspension aqueuse des fibres, soit par un traitement en surface de la feuille humide, afin de diminuer l'absorption de l'eau par la feuille. Ensuite on sèche la feuille.

Cette feuille peut être enduite sur l'une de ses faces, uniformément, d'une couche continue ou selon des motifs notamment de grilles ou de zones, d'un adhésif scellant à chaud et/ou sous pression. Ensuite elle est scellée contre un film plastique ou une autre feuille de papier barrière aux micro-organismes, sur un certain périmètre selon la forme voulue pour l'emballage tout en laissant une ouverture. Dans certains cas la feuille de papier n'est pas enduite d'un tel adhésif, car grâce à sa composition comprenant un composé ayant des propriétés thermoscellantes comme l'amidon par exemple ou encore un polymère thermoplastique sous forme de fibres ou introduit sous forme d'une émulsion aqueuse stable (latex), elle peut être thermoscellée directement contre un film thermoplastique.
Les emballages sont découpés au format adéquat.

La perméabilité spécifique de ces feuilles de papier est obtenue par un choix des pâtes cellulosiques, comprenant en général entre 0 et 70 % en poids de fibres courtes et le complément à 100 en fibres longues, de leur raffinage (non raffinées à un raffinage de 40 degrés Schoepper-Riegler) et par des réglages sur la machine à papier connus de l'homme du métier. L'enchevêtrement des fibres de la feuille permet d'avoir un compromis entre la porosité de la feuille et le diamètre des pores qui crée cette perméabilité spécifique nécessaire et un chemin tortueux pour ne laisser pénétrer les molécules de vapeur d'eau ou de gaz stérilisants sans laisser traverser les poussières porteuses de bactéries ou autres micro-organismes. Dans le domaine des emballages de stérilisation scellables, on recommande une moyenne du diamètre de pores équivalent ne dépassant pas 35 µm et qu'aucune valeur individuelle de diamètre ne dépasse 50 µm, selon la norme BS 3321 :1986.

Dans la demande de brevet anglais GB 1559843, on a décrit un sachet de stérilisation formé d'un côté d'un film thermoplastique imperméable (1) et de l'autre côté d'un complexe (3) feuille de papier/film thermoplastique imperméable scellé à une feuille de papier (2) barrière aux bactéries et perméable aux agents stérilisants (gaz ou vapeur d'eau), le complexe étant d'une longueur inférieure aux autres constituants de façon à ce que les agents stérilisants puissent pénétrer dans la zone de la feuille (2) restant perméable. La constitution de cet emballage a pour but de protéger les objets stérilisés lors de l'ouverture du sachet. Il empêche en particulier que des particules qui pourraient être arrachées de la feuille de papier (2) lors de l'ouverture, se déposent sur les objets. Il présente cependant l'inconvénient d'être peu pratique dans sa fabrication car il faut assembler des feuilles de longueur différentes et les agents stérilisants ne peuvent pénétrer que sur une zone réduite, ce qui peut être préjudiciable à une bonne stérilisation.

Par ailleurs on connaît des feuilles pour des emballages de stérilisation scellables à base de fibres uniquement de cellulose (sans fibres synthétiques) qui sont fabriquées et commercialisées par ARJO WIGGINS sous les marques ETHYPEL ® et PROPYPEL ® en Europe. Ces feuilles ont une barrière bactérienne élevée mais ont une résistance mécanique qui peut s'avérer insuffisante même si elles ont été traitées en surface par agent renforçant comme l'amidon, le polyalcool de vinyle ou un latex acrylique, lorsque l'on souhaite emballer des objets lourds ou contondants.

On a alors proposé des feuilles papetières renforcées par des fibres synthétiques en mélange avec les fibres de cellulose. Par exemple de telles feuilles comportant des fibres synthétiques de polyester sont vendues par ARJO WIGGINS sous la marque STERISHEET ® en Europe . A grammage équivalent, ces feuilles dites renforcées ont une résistance mécanique supérieure aux feuilles purement cellulosiques mais en revanche leur barrière bactérienne est un peu moins élevée.

On a proposé aussi d'autres feuilles d'emballage de stérilisation qui sont des feuilles de non-tissés obtenus par voie sèche et ne comportant que des fibres synthétiques liées à chaud. Par exemple de telles feuilles en fibres de polyéthylène sont commercialisées sous la marque TYVEK ® par la société Du Pont de Nemours. Ces feuilles présentent une grande résistance mécanique. Cependant un inconvénient de ces feuilles est que leur épair est très hétérogène, c'est-à-dire que la répartition des fibres est très irrégulière et donc que la perméabilité de la feuille n'est pas uniforme. Ainsi, à certains endroits, la feuille peut présenter des pores de diamètre trop important.
Ces feuilles purement synthétiques ont de plus l'inconvénient d'avoir des temps de biodégradabilité extrêmement longs. Par ailleurs elles sont plus coûteuses.

On recherche donc à ce qu'une feuille d'emballage de stérilisation présente simultanément et obligatoirement plusieurs propriétés.

L'une des propriétés est que la feuille doit être résistante à la déchirure. En effet, comme elle est destinée à faire des emballages qui vont contenir des objets qui peuvent être lourds ou contondants, elle risque d'être déchirée ou transpercée par ces objets lors de la manipulation desdits emballages. Dans le cas d'un emballage de stérilisation scellable, on recherche une résistance moyenne à la déchirure supérieure à 300 mN mesurée selon la norme européenne EN 21974.

Une autre propriété mécanique est que la feuille doit être résistante à l'éclatement. En effet , lors de la stérilisation des emballages, elle peut être soumise à une pression lors de l'injection des gaz stérilisants et à une forte dépression postérieure lors de l'évacuation des gaz qui doit être complète. Dans le cas d'un emballage de stérilisation scellable, on recherche une résistance à l'éclatement supérieure ou égale à 200 kPa mesurée selon la norme ISO 2758.

De plus, une autre propriété mécanique de la feuille est sa résistance à un impact. Dans le cas d'un emballage de stérilisation scellable, on recherche une résistance, caractérisée par sa résistance à l'impact d'un pendule déterminée selon la norme ASTM D3420, qui est supérieure ou ègale à 0,40 Joule.

Une autre propriété est que la feuille doit être perméable aux agents stérilisants. En effet, comme décrit précédemment, lorsqu'on a introduit l'objet dans l'emballage, puis que l'on a scellé celui-ci, on soumet l'ensemble à l'action de gaz stérilisants ou de vapeur d'eau. Dans le cas d'un emballage de stérilisation scellable, on recherche une perméabilité à l'air mesurée selon la norme ISO 5636-3, méthode BENDTSEN supérieure à 0,2 µm/(Pa.s).

Cependant une autre propriété est que la feuille doit être barrière aux bactéries ou autres micro-organismes pour maintenir la stérilité de l'emballage c'est-à-dire qu'il ne faut pas que les micro-organismes puissent pénètrer à l'intérieur de l'emballage après la stérilisation. Il faut donc que la moyenne des diamètres les plus élevés des pores ne soit pas trop importante et qu'il n'y ait pas de pores avec un diamètre trop élevé. Cette propriété barrière peut être caractérisée par l'efficacité en filtration bactérienne, nommée couramment sous les initiales de sa terminologie anglaise BFE (Bacterial Filtration Efficiency) ; elle est exprimée en un pourcentage qui représente le pourcentage de bactéries arrêtées par la feuille. Dans le cas d'un emballage de stérilisation scellable, on recherche une BFE d'au moins 85 %.

Une autre propriété est que cette feuille soit scellable soit par sa composition soit par son aptitude à recevoir une couche scellante.

Une autre propriété recherchée pour ces feuilles est qu'elles permettent une ouverture aseptique de l'emballage après stérilisation. Cette ouverture consiste en ce que, lorsqu'on ouvre l'emballage stérilisé, il n'y ait pas de fibres ou d'autres particules de la feuille qui se détachent et viennent se déposer sur les objets stérilisés. Pour cela l'ouverture de l'emballage doit se faire sans arracher la feuille. On dit que les feuilles sont pelables.

Une autre propriété recherchée pour l'emballage obtenu est que les forces de scellage doivent être suffisantes pour éviter que l'emballage ne s'ouvre malencontreusement.
Donc, plus les dispositifs à stériliser sont lourds, plus la force de scellage de l'emballage doit être importante afin que l'emballage ne s'ouvre pas sous leur poids. Or plus on augmente cette force, plus les risques d'arrachage de la feuille à l'ouverture sont élévées. Il faut donc que la feuille ait aussi une très forte cohésion de surface et/ou interne pour avoir une bonne ouverture aseptique.

Il est donc nécessaire que les feuilles d'emballage de stérilisation soient à la fois résistantes mécaniquement, perméables aux gaz stérilisants et barrières aux micro-organismes.

En augmentant le grammage des feuilles, il est possible d'améliorer certaines de ces caractéristiques sans toutefois obtenir l'ensemble de toutes les caractéristiques recherchées et de plus cela augmente leur coût. Par ailleurs, si on essaie de renforcer la surface des feuilles pour avoir par exemple une plus forte cohésion, en l'imprégnant d'un produit renforçant, on ouvre sa structure et on diminue alors son efficacité en filtration bactérienne. Des essais faits sur la feuille de l'exemple 5 comparatif mentionné plus loin ont montré ce fait.

Le problème est donc de fournir une feuille d'emballage de stérilisation qui présente les propriétés requises exposées ci-avant, en particulier qui présente à la fois une très grande résistance mécanique globale et une haute barrière microbienne, et ce, tout en minimisant son grammage.

La Demanderesse a trouvé qu'en liant deux feuilles d'emballage de stérilisation entre elles par l'une de leurs faces de façon non réversible, notamment par contrecollage, on obtient un matériau qui répond au problème car il présente toutes les caractéristiques recherchées et de plus ledit matériau est globalement supérieur à une feuille simple d'emballage de stérilisation qui aurait le même grammage que ledit matériau.

Ainsi l'invention fournit un matériau d'emballage de stérilisation scellable pour les dispositifs médicaux devant être stérilisés, ayant une résistance moyenne à l'éclatement supérieure ou égale à 200 kPa mesurée selon la norme ISO 2758, une résistance moyenne à la déchirure supérieure ou égale à 300 mN mesurée selon la norme européenne EN 21974, une résistance moyenne à l'impact mesurée selon la norme ASTM D3420 supérieure ou égale à 0,4J et une efficacité en filtration bactérienne BFE supérieure ou égale à 85 %, ayant un grammage compris entre 40 et 250 g/m², de préférence entre 70 et 250 g/m², mesuré selon la norme ISO 536, et qui comporte au moins deux feuilles d'emballage de stérilisation scellables (F1) et (F2), dont au moins l'une des feuilles est scellable directement ou après enduction d'un produit scellant, lesdites feuilles étant liées entre elles par l'une de leurs faces de façon non réversible.
On entend par le fait que les deux feuilles sont liées de façon non réversible que les deux feuilles ne peuvent pas être séparées sans que l'une au moins soit déchirée c'est-à-dire que la force de liaison entre les deux feuilles est supérieure à la plus faible des forces de cohésion des feuilles.

De préférence, le matériau selon l'invention a une efficacité en filtration bactérienne BFE supérieure ou égale à 90% et encore de préférence à 95 %.

Plus particulièrement l'invention fournit un matériau qui se caractérise par le fait que les deux feuilles d'emballage de stérilisation (F1) et (F2) sont liées par des points de liaison tel que lesdits points de liaison soient sous forme discrète à l'interface de liaison des feuilles.

De préférence le matériau se caractérise par le fait que les points de liaison discrets sont uniformément répartis à l'interface des feuilles.

Les feuilles (F1) et (F2) peuvent être liées par toute technique permettant que le matériau résultant de l'assemblage des feuilles ait une bonne perméabilité aux agents stérilisants et une bonne barrière aux micro-organismes. Par conséquent, il ne faut pas que l'interface des feuilles assemblées soit trop bouchante. Il peut s'agir de toute technique permettant de faire des points de liaison discrets entre les feuilles comme le liage à chaud, par ondes radio hautes fréquences, ultra-sons ou par un moyen de dépôt adapté d'un adhésif ou encore par usage d'un adhésif poreux.

Selon un mode préféré de l'invention, le matériau se caractérise par le fait que les feuilles (F1) et (F2) sont liées par un adhésif.

Selon un mode de réalisation particulier de l'invention, l'adhésif est déposé par un procédé de couchage par héliogravure à cylindre tramé c'est-à-dire que le cylindre a une trame avec des cellules espacées régulièrement ou selon une grille ou d'autres motifs.

L'adhésif peut être une colle usuellement utilisée dans le domaine du contrecollage papetier comme l'amidon, certains polymères utilisés sous forme d'émulsion aqueuse stable comme en particulier les polyacrylates, les polyuréthanes, les copolymères styrène-butadiène éventuellement carboxylés ; il peut s'agir d'un adhésif sensible à la pression , connu sous la dénomination d'adhésif PSA ou d'un adhésif fusible à chaud comme les adhésifs dits hot-melts.

Ainsi , selon un cas particulier de l'invention, l'adhésif est choisi parmi les adhésifs sensibles à la pression.

Selon un autre cas particulier de l'invention, l'adhésif est choisi parmi les adhésifs fusibles à chaud, dits hot-melts.

Selon un cas particulier de l'invention, l'adhésif est un adhésif autoscellable, en particulier le caoutchouc naturel.

En effet, avantageusement on peut utiliser un adhésif comme le caoutchouc naturel, le polyisoprène cis 1-4, qui a la particularité de ne coller que sur lui-même et donc de pouvoir facilement bobiner des feuilles qui en sont enduites. On peut alors coller ensemble deux feuilles ainsi déjà enduites en ligne sur la machine de fabrication en particulier d'une machine à papier sans avoir à déposer ultérieurement un adhésif, en mettant la face enduite d'une feuille contre la face enduite de l'autre feuille. L'assemblage des feuilles peut se faire sur la machine de fabrication si elle possède l'équipement adéquat.

Selon un cas particulier de l'invention, l'adhésif est un adhésif poreux. Cet adhésif poreux peut être préparé en créant des pores dans un adhésif connu soit par l'action d'une réaction chimique produisant un gaz et ce avant, pendant, après le dépôt de l'adhésif, soit en injectant un gaz inerte ou de l'air dans l'adhésif avant ou pendant son dépôt sur l'une des feuilles.

De plus l'adhésif, comme tous les autres constituants du matériau, doit être sélectionné sur des critères de non toxicité compte-tenu de la destination du matériau, en s'appuyant par exemple sur la norme ISO 10993-5 relative à la caractérisation de non cytotoxicité d'un matériau.

Cet adhésif peut avoir à la fois des caractéristiques d'une colle et aussi un caractère renforçant mécaniquement pour les feuilles donc pour le matériau.

L'adhésif peut être déposé sur la surface de l'une des feuilles ou de chacune des feuilles.

La quantité d'adhésif déposée sera la plus faible possible mais devra assurer une liaison permanente entre les feuilles dans des conditions liées à la destination du matériau, en particulier après avoir été soumis à la stérilisation.

Préférentiellement la quantité d'adhésif déposée sera comprise entre 1 et 20 g/m² et plus particulièrement entre 5 et 10 g/m².

La feuille (F1) peut être une feuille obtenue par voie papetière comportant des fibres uniquement cellulosiques, éventuellement modifiées comme les fibres de rayonne issues du procédé sodique de la viscose ou des fibres de cellulose régénérées en milieu solvant comme celles commercialisées sous les marques Lyocell ® ou Tencel ®, en mélange avec un agent de résistance humide et un agent de renforcement papetier ajouté en masse ou en surface comme un alcool polyvinylique , un amidon ou un polymère ajouté sous forme d'une émulsion aqueuse stable (latex) notamment les polymères acryliques ou acrylates.

La feuille (F1) peut être aussi une feuille obtenue par voie papetière comportant des fibres de cellulose, éventuellement modifiée comme les fibres de rayonne issues du procédé sodique de la viscose ou des fibres de cellulose régénérées en milieu solvant comme celles commercialisées sous les marques Lyocell ® ou Tencel ®, en mélange avec des fibres synthétiques, toutes ces fibres étant liées soit par thermoliage, soit par hydroliage ou soit par voie chimique grâce à l'ajout en masse, ou grâce à un traitement de surface comme en presse encolleuse ou par pulvérisation, d'un agent liant utilisé habituellement en papeterie comme un alcool polyvinylique , un amidon ou un polymère ajouté sous forme d'une émulsion aqueuse stable (latex) . En particulier les fibres synthétiques sont en quantités comprises entre 5 et 95 parts en poids sec, la somme totale des fibres faisant 100 parts.

Ainsi l'invention fournit un matériau qui se caractérise par le fait que la feuille (F1) est une feuille papetière et qu'elle comporte :
- entre 5 et 100 parts en poids de fibres de cellulose, éventuellement modifiée,
- entre 0 et 95 parts en poids de fibres synthétiques, la somme des parts en fibres cellulosiques et synthétiques faisant 100,
- entre 0 et 5 % d' un agent de résistance humide, en poids sec de la composition totale de la feuille ,
- entre 0 et 40 % , en poids sec de la composition totale de la feuille, d'un agent de cohésion.

Les feuilles peuvent être éventuellement crêpées, microcrêpées ou embossées à sec. Elles peuvent être colorées ou nuancées.

On peut utiliser aussi des feuilles ne comportant que des fibres synthétiques en particulier de type non-tissés, cependant on préfère celles comportant au moins en partie des fibres cellulosiques car elles ont une meilleure biodégradabilité.

La feuille (F2) peut être une feuille obtenue par voie papetière comportant des fibres uniquement de cellulose, éventuellement modifiée comme les fibres de rayonne issues du procédé sodique de la viscose ou des fibres de cellulose régénérées en milieu solvant comme celles commercialisées sous les marques Lyocell ® ou Tencel ®, en mélange avec un agent de résistance humide.

La feuille (F2) peut être aussi une feuille comportant les mêmes composés que les feuilles (F1) décrites ci-dessus avec de préférence comme fibres synthétiques des microfibres qui apportent un meilleur niveau en filtration microbienne.

En particulier l'invention fournit un matériau qui se caractérise par le fait que la feuille (F2) est une feuille papetière et qu'elle comporte :
- entre 90 et 100 parts en poids de fibres de cellulose, éventuellement modifiée,
- entre 0 et 10 parts en poids de fibres synthétiques, la somme des parts en fibres cellulosiques et synthétiques faisant 100 ;
- entre 0 et 5 % d' un agent de résistance humide, en poids sec de la composition totale de la feuille,
- entre 0 et 40 % , en poids sec de la composition totale de la feuille, d'un agent de cohésion.

De préférence les fibres synthétiques sont choisies parmi les fibres d'homopolymères ou de copolymères d'oléfines, de polyester, de polyamide et leurs mélanges. Ces fibres peuvent être aussi des fibres bicomposantes ayant un coeur et une enveloppe de natures chimiques différentes et/ou de caractéristiques différentes comme par exemple leurs points de fusion. Ces fibres peuvent être des fibres coupées.

Les fibres synthétiques ont de préférence une longueur comprise entre 1 et 30 mm et par ailleurs un titre compris enre 0,4 et 5 dtex.

Selon un mode particulier de l'invention, l'agent de cohésion est aussi l'adhésif de liaison entre les feuilles (F1) et (F2).

Le matériau obtenu pourra alors être utilisé pour faire des emballages scellables destinés à la stérilisation de dispositifs médicaux selon les mises en oeuvre connues dans ce domaine et par exemple comme exposées plus haut dans la description de l'art antérieur.

Ainsi l'invention fournit un emballage de stérilisation scellable pour les dispositifs médicaux devant être stérilisés qui se caractérise par le fait qu'il comporte ledit matériau d'emballage de stérilisation.

En particulier, l'invention fournit un emballage qui se caractérise par le fait que la feuille (F2) ayant la plus grande efficacité en filtration bactérienne BFE est située à l'extérieur de l'emballage.

Selon un mode particulier, l'invention fournit un emballage qui se caractérise par le fait qu'il est constitué par ledit matériau de stérilisation et un film en polymère thermoplastique imperméable aux gaz qui est scellé contre ledit matériau sur une partie de son périmètre.

Selon un autre mode particulier, l'invention fournit un emballage qui se caractérise par le fait qu'il est constitué par ledit matériau de stérilisation scellé contre lui- même ou une feuille de papier enduite d'un produit scellant comme une couche de polyéthylène extrudé ou du poly (acétate de vinyle).

Selon un autre mode particulier, l'invention fournit un emballage qui se caractérise par le fait qu'il est constitué d'un container rigide et d'un opercule formé par ledit matériau de stérilisation.

Bien que préférentiellement l'invention concerne le liage de deux feuilles, elle n'est pas limitée à l'utilisation de deux feuilles seulement, l'homme du métier saura adapter les grammages et caractéristiques des différentes feuilles selon l'enseignement général de la présente description.

L'invention sera mieux comprise à l'aide d'exemples selon l'invention non limitatifs et des exemples comparatifs décrits ci-dessous.

### EXEMPLE 1 selon l'invention :

- Réalisation d'une feuille d'emballage (F1) :
   On réalise la feuille sur une machine à papier Foudrinier.
   On met en suspension en milieu aqueux des fibres de cellulose et des fibres synthétiques de polyester dans des proportions respectives de 90 parts et 10 parts en poids sec. Les fibres de cellulose sont un mélange de 20 % en poids de fibres courtes et de son complément à 100 en fibres longues, les fibres étant raffinées à 25 °SR. Les fibres de polyester ont une longueur comprise entre 5 et 25 mm et un titre de 1,7 dtex. A cette suspension, on ajoute 0,26 % en poids sec de la composition totale de la feuille, d'un agent de résistance humide de type PAE (polyamine epichlorhydrine) et 1 % en poids sec de la composition totale de la feuille, d'un amidon cationique comme agent de cohésion interne.
   On égoutte cette suspension sur la toile de la machine pour former la feuille.
   On imprègne la feuille en presse encolleuse d'un agent de cohésion synthétique acrylique introduit sous forme d'une émulsion aqueuse stabilisée. Cet agent acrylique est présent à raison de 8 g/m² en poids sec.
   On séche la feuille vers 120 °C.
   La feuille a alors un grammage de 47,4 g/m².
- Réalisation d'une feuille d'emballage (F2) :
   On réalise la feuille sur une machine à papier Foudrinier.
   On met en suspension en milieu aqueux des fibres de cellulose. Les fibres de cellulose sont un mélange de 20 % en poids de fibres courtes et de son complément à 100 ( soit 80 %) en fibres longues, les fibres étant raffinées à 25 °SR. A cette suspension, on ajoute 0,26 % en poids sec de la composition totale de la feuille, d'un agent de résistance humide de type PAE (polyamine epichlorhydrine), 0,15 % en poids sec de la composition totale de la feuille, d'un agent de collage de type dit AKD (dimère d'alkylcétène) et 1 % en poids sec de la composition totale de la feuille, d'un amidon cationique comme agent de cohésion interne.
   On égoutte cette suspension sur la toile de la machine pour former la feuille.
   On imprègne la feuille en presse encolleuse d'un agent de cohésion soluble dans l'eau qui est un amidon. Cet agent est présent à raison de 0,5 g/m² en poids sec de la composition totale de la feuille.
   On séche la feuille vers 120 °C.
   La feuille a alors un grammage de 61,3 g/m².
- Contrecollage des feuilles (F1) et (F2) pour former le matériau selon l'invention :
   On dépose sur la face de l'une des feuilles une colle à base de copolymères vinyliques en milieu aqueux. La colle est déposée à raison de 5,3 g/m², par un système de couchage par héliogravure à cylindre tramé. On lie les deux feuilles précédemment fabriquées par passage entre des rouleaux. On séche la feuille vers 150°C.
   On bobine le matériau obtenu.
   Le grammage du matériau est de 114 g/m².
- Réalisation de l'emballage de stérilisation :
   Par héliogravure, on enduit le matériau du côté de la feuille (F1) avec une laque thermoscellante à base d'un copolymère acétate de vinyle - éthylène en milieu aqueux, à raison de 4 g/m² en poids sec. On sèche le matériau enduit. On bobine.
   Sur une soudeuse-découpeuse, on assemble par thermoscellage le matériau enduit et un film thermoplastique de polyéthylène de façon à former des sachets scellés sur trois côtés et un côté ouvert. Ensuite les sachets sont découpés.
   Les sachets pourront être utilisés pour stériliser des dispositifs médicaux qu'on y aura introduits.

### EXEMPLE 2 selon l'invention :

- Réalisation d'une feuille d'emballage (F1) :
   On réalise la feuille sur une machine à papier Foudrinier.
   On met en suspension en milieu aqueux des fibres de cellulose. A cette suspension, on ajoute 0,26 %, en poids sec de la composition totale de la feuille, d'un agent de résistance humide de type PAE (polyamine epichlorhydrine), 0,10% en poids sec de la composition totale de la feuille, d'un agent de collage de type dit AKD (dimère d'alkylcétène) et 1 %, en poids sec de la composition totale de la feuille, d'un amidon cationique comme agent de cohésion interne.
   On égoutte cette suspension sur la toile de la machine pour former la feuille.
   On imprègne la feuille en presse encolleuse d'un agent de cohésion synthétique acrylique introduit sous forme d'une émulsion aqueuse stabilisée. Cet agent acrylique est présent à raison de 8 g/m² en poids sec.
   On séche la feuille vers 120 °C.
   La feuille a alors un grammage de 42,4 g/m².
- Réalisation d'une feuille d'emballage (F2) : on réalise la même feuille d'emballage (F2) que dans l'exemple 1.
- Contrecollage des feuilles (F1) et (F2) pour former le matériau selon l'invention :
   On dépose sur la face de l'une des feuilles une colle à base de copolymères vinyliques en milieu aqueux. La colle est déposée à raison de 10,3 g/m², par un système de couchage par héliogravure à cylindre tramé. On lie les deux feuilles précedemment fabriquées par passage entre des rouleaux. On séche la feuille vers 150°C.
   On bobine le matériau obtenu.
   Le grammage du matériau est de 114 g/m².
- Réalisation de l'emballage de stérilisation : on réalise un emballage comme dans l'exemple 1.

### EXEMPLE 3 :

- Réalisation d'une feuille d'emballage (F1) :
   On réalise la feuille sur une machine à papier Foudrinier.
   On met en suspension en milieu aqueux des fibres de cellulose. A cette suspension, on ajoute 0,26 %, en poids sec de la composition totale de la feuille, d'un agent de résistance humide de type PAE (polyamine epichlorhydrine), 0,12 % en poids sec de la composition totale de la feuille, un agent de collage de type dit AKD (dimère d'alkylcétène) et 1 %, en poids sec de la composition totale de la feuille, d'un amidon cationique comme agent de cohésion interne.
   On égoutte cette suspension sur la toile de la machine pour former la feuille.
   On imprègne la feuille en presse encolleuse d'un agent de cohésion synthétique acrylique introduit sous forme d'une émulsion aqueuse stabilisée. Cet agent acrylique est présent à raison de 4 g/m² en poids sec.
   On séche la feuille vers 120 °C.
   La feuille a alors un grammage de 45,4 g/m².
   On réalise un microcrêpage de la feuille en la crèpant à sec.
- Réalisation d'une feuille d'emballage (F2) : on reprend la feuille (F2) décrite à l'exemple 1.
- Contrecollage des feuilles (F1) et (F2) pour former le matériau selon l'invention :
   Les feuilles (F1) et (F2) sont liées comme dans l'exemple 2 avec une quantité de 6,3 g/m² de colle déposée.

### EXEMPLE 4 comparatif :

Cet exemple consiste en une feuille d'emballage de stérilisation, destinée à l'enduction par un produit scellant, qui comprend, à notre connaissance, des fibres purement cellulosiques et un haut taux de polymère introduit sous forme d'une dispersion aqueuse (latex) à la fois en masse et en surface de la feuille. Cette feuille est fabriquée et commercialisée en un grammage de 115 g/m² par la société KIMBERLY CLARK.

### EXEMPLE 5 comparatif :

On réalise une feuille sur une machine à papier Foudrinier.
On met en suspension en milieu aqueux des fibres de cellulose. A cette suspension, on ajoute 0,26 %, en poids sec de la composition totale de la feuille, d'un agent de résistance humide de type PAE (polyamine epichlorhydrine), 0,17 % en poids sec de la composition totale de la feuille, d'un agent de collage de type dit AKD (dimère d'alkylcétène) et 1 %, en poids sec de la composition totale de la feuille, d'un amidon cationique comme agent de cohésion interne.
On égoutte cette suspension sur la toile de la machine pour former la feuille.
On imprègne la feuille en presse encolleuse d'un mélange d'amidon et d'agent de collage. Ce mélange est présent à raison de 1 g/m² en poids sec.
Son grammage est de 115 g/m².

### RESULTATS :

Les mesures déterminées selon les méthodes exposées plus loin, sur les échantillons des exemples 1 et 4 et 5, sont présentées dans le tableau 1 et celles pour les exemples 2 et 3 dans le tableau 2. La perméabilité de la feuille (F1) de l'exemple 3 n'est pas donnée car cette feuille étant crêpée, elle n'est pas déterminable par la méthode BENDTSEN.

Ces résultats montrent que d'une part l'efficacité en barrière bactérienne et d'autre part la résistance mécanique globale et en particulier la résistance à l'impact ainsi que la résistance à l'arrachage sont meilleures pour les matériaux selon l'invention que pour de simples feuilles d'emballage de stérilisation de même grammage.

En particulier, dans le tableau 1, on mentionne les caractéristiques physiques et bactériostatiques des feuilles et matériaux des exemples 1, 4 et 5 , avant et après stérilisation aux rayonnements gamma.
L'échantillon à tester a été soumis à une source de Cobalt 60, source de rayonnements ionisants, dits gamma. La dose de rayonnements absorbés par l'échantillon est de 50 kGy.
Il est connu que les caractéristiques sont altérées par la stérilisation par rayonnements, en particulier pour les produits à base de cellulose, néanmoins, le tableau 1 montre que le complexe selon l'invention conserve des caractéristiques de niveaux acceptables.

### EXEMPLES 6 à 10 :

On réalise des matériaux contrecollés de grammages différents, entre 70 et 260 g/m², de la même façon que celle décrite à l'exemple 1, en utilisant des feuilles F1 et F2 ayant respectivement les compositions de celles de l'exemple 1 mais de grammages variables. La quantité de la colle de contrecollage est quasi-constante et est d'eviron 5,5 g/m² en sec.
Les caractéristiques physiques et bactériostatiques des feuilles F1 et F2 et des matériaux F1/F2 obtenus sont données dans le tableau 3.
Ce tableau 3 montre que toutes les caractéristiques pour le matériau F1/F2 sont élévées par rapport aux caractéristiques des feuilles de bases et, en particulier, il apparaît que la résistance à l'arrachage est nettement améliorée.

### EXEMPLES 11 à 14 :

On réalise des matériaux contrecollés dans un grammage donné, de la même façon que celle décrite à l'exemple 1, en utilisant des feuilles F1 et F2 ayant respectivement les compositions de celles de l'exemple 1 et on fait varier la quantité de la colle de contrecollage déposée entre 1,5 et 20 g/m² en sec.
Les caractéristiques physiques et bactériostatiques des feuilles F1 et F2 et des matériaux F1/F2 obtenus sont données dans le tableau 4.
Ce tableau 4 montre que toutes les caractéristiques pour le matériau F1/F2 sont élévées par rapport aux caractéristiques des feuilles de bases et, en particulier, il apparaît que la résistance à l'arrachage est nettement améliorée.

### METHODES DE CARACTERISATION:

Les feuilles (F1) et (F2) et les matériaux obtenus ont été caractérisés par les méthodes référencées ci-dessous.
Les mesures, sauf la BFE, ont été faites sur des échantillons conditionnés selon la norme européenne EN 20187 (équivalente à la norme ISO 187 : 1995) selon laquelle la température doit être maintenue à 23 °C et l'humidité relative à 50 %.
Les mesures sont la moyenne des mesures déterminées sur chaque face des échantillons.
Le grammage est déterminé selon la norme internationale ISO 536.
La résistance moyenne à la déchirure (sens marche et sens travers) est mesurée selon la norme européenne EN 21974 et qui correspond à la norme internationale ISO-1974:1990 (méthode Elmendorf).
La résistance moyenne à l'éclatement à sec est mesurée selon la norme ISO 2758.
La résistance moyenne à l'impact par pendule est exprimée par une énergie de rupture déterminée selon la norme américaine ASTM D3420 sur un appareil de marque SPENCER, avec un pendule 800. Quand la valeur limite déterminable par le matériel est atteinte, on a mentionné dans le tableau « supérieure à ».
La perméabilité moyenne à l'air est mesurée selon la norme ISO 5636/3 (méthode Bendtsen). Cette méthode ne s'applique pas aux feuilles crêpées.
La moyenne du diamètre des pores équivalent est mesurée selon la norme anglaise BS 3321 :1986.
L'efficacité en filtration bactérienne BFE est déterminée selon la méthode publiée par l'association américaine EDANA sous la référence 180.0-89 de février 1996 .
La bonne cohésion du matériau pour avoir l'aptitude à l'ouverture aseptique (pelabilité) est déterminée par le test de résistance à l'arrachage au ruban adhésif. Ce test est effectué en appliquant un ruban adhésif ayant une largeur comprise entre 1,27 et 1,90 cm sur la face de la feuille F1 du complexe F1/F2. On scelle le ruban adhésif à 116 °C et sous une pression de 278 kPa pendant 2 secondes. On laisse refroidir puis on pèle le ruban à vitesse constante sous un angle de 180 degrés. On apprécie visuellement l'arrachage des particules sur le ruban adhésif.

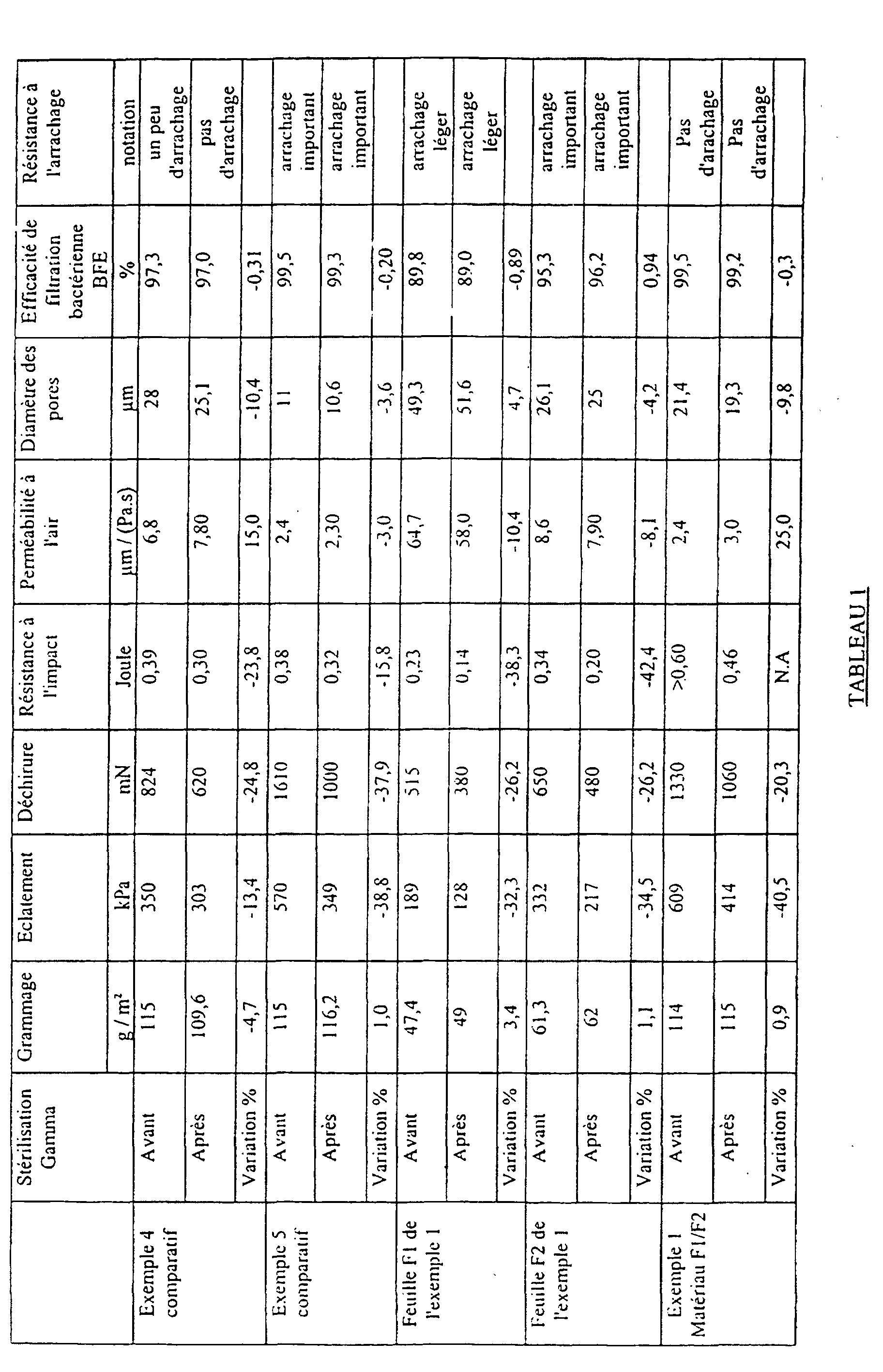

**TABLEAU 2**

| | EXEMPLE 2 | | | EXEMPLE 3 | | |
|---|---|---|---|---|---|---|
| | Feuille F1 | Feuille F2 | Matériau F1/F2 | Feuille F1 | Feuille F2 | Matériau F1/F2 |
| Grammage (g/m²) | 42,4 | 61,3 | 114 | 45,4 | 61,3 | 113 |
| Résistance à l'éclatement (kPa) | 200 | 331,5 | 480 | 150 | 331,5 | 405,6 |
| Résistance à la déchirure (mN) | 400 | 650 | 1250 | 440 | 650 | 1214 |
| Résistance à l'impact (J) | 0,21 | 0,34 | >0,590 | 0,210 | 0,344 | 0,439 |
| Perméabilité à l'air [µm/(Pa.s)] | 37,7 | 8,6 | 1,58 | - | 8,6 | 3,1 (face lisse) |
| Diamètre des pores (µm) | 36 | 26,1 | 21,9 | 40,4 | 26,1 | 23,9 |
| Efficacité de filtration bactérienne "BFE" (%) | 90,1 | 95,3 | 99,6 | 86,1 | 95,3 | 98,9 |
| TEST de résistance à l'arrachage | | | Pas d'arrachage | | | Pas d'arrachage |

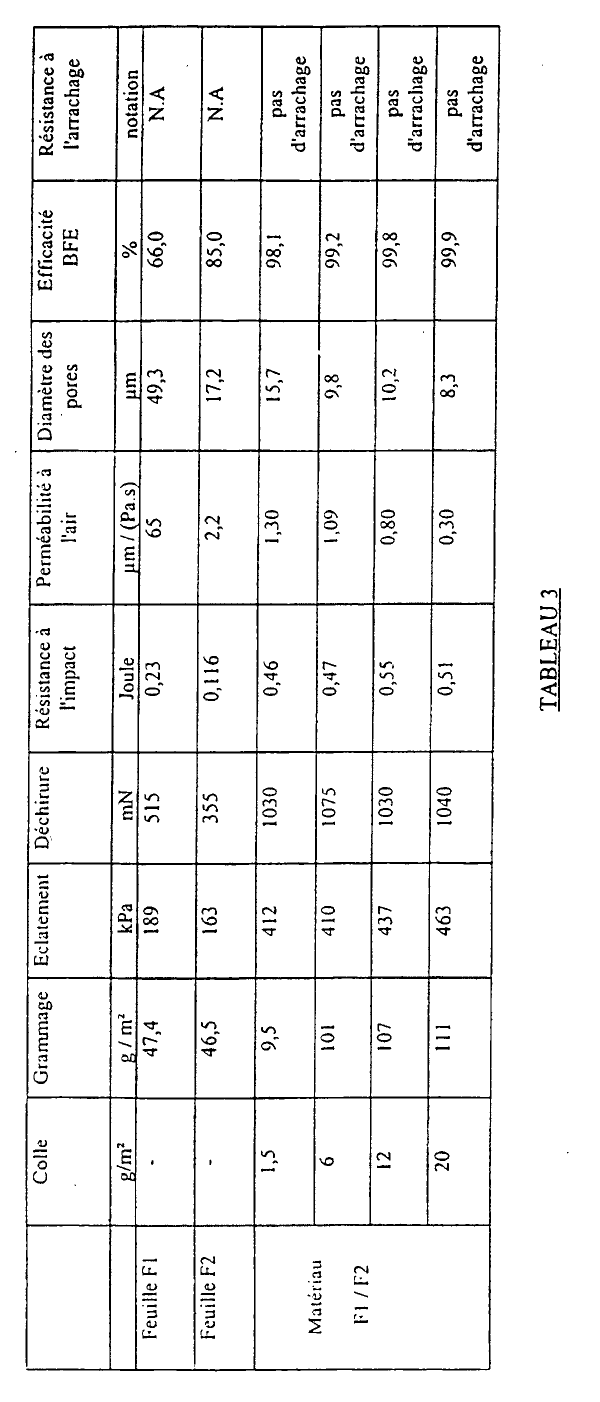

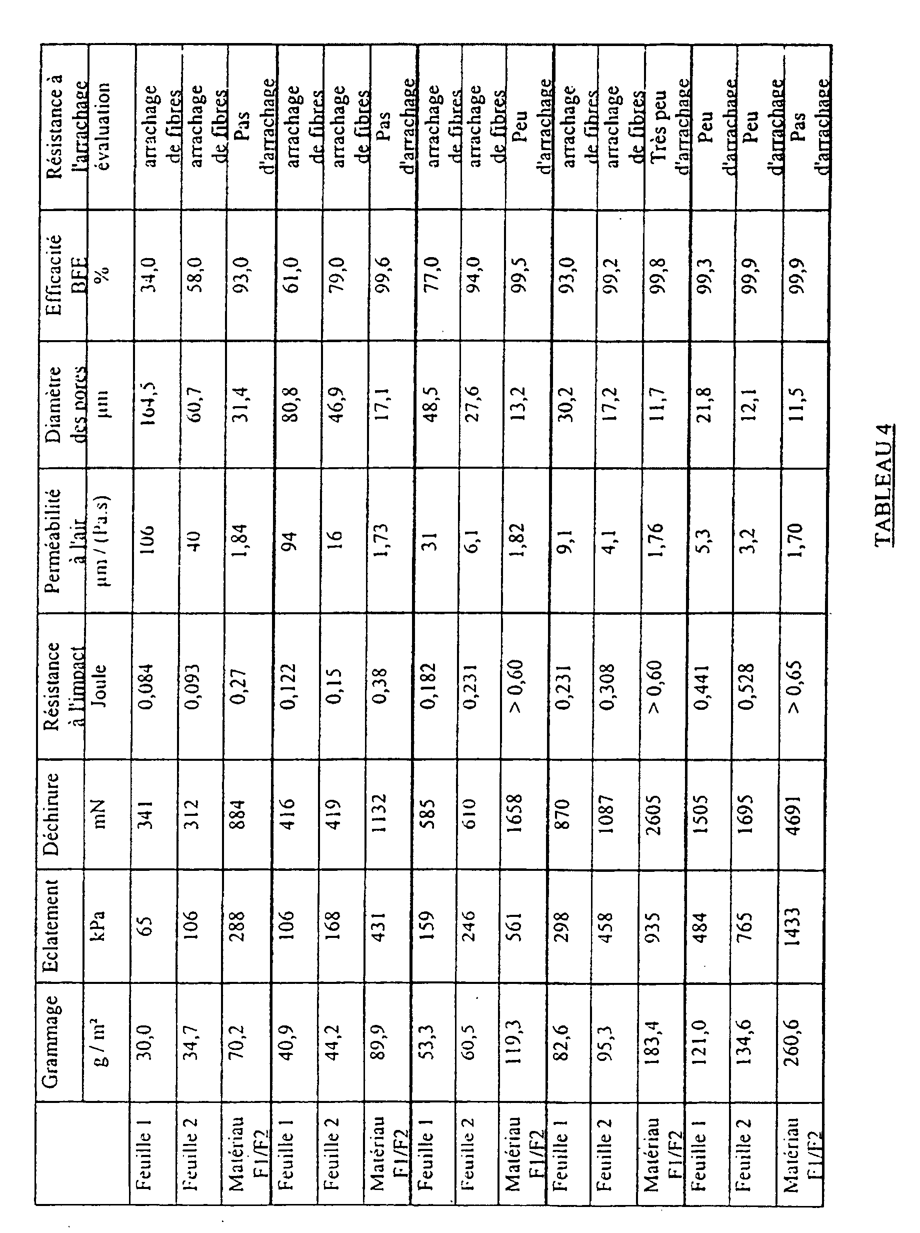

## Revendications

1. Matériau d'emballage de stérilisation scellable pour les dispositifs médicaux devant être stérilisés, ayant une résistance moyenne à l'éclatement supérieure ou égale à 200 kPa mesurée selon la norme ISO 2758, une résistance moyenne à la déchirure supérieure ou égale à 300 mN mesurée selon la norme européenne EN 21974, une résistance moyenne à l'impact mesurée selon la norme ASTM D3420 supérieure ou égale à 0,4J et une efficacité en filtration bactérienne BFE supérieure ou égale à 85 %, ayant un grammage compris entre 40 et 250 g/m², de préférence entre 90 et 250 g/m², mesuré selon la norme ISO 536, et qui comporte au moins deux feuilles d'emballage de stérilisation (F1) et (F2), l'une au moins étant scellable directement ou après enduction d'un produit scellant, lesdites feuilles étant liées entre elles par l'une de leurs faces de façon non réversible.

2. Matériau selon la revendication 1, **caractérisé par le fait que** les deux feuilles d'emballage de stérilisation (F1) et (F2) sont liées par des points de liaison tel que lesdits points de liaison soient sous forme discrète à l'interface de liaison des feuilles.

3. Matériau selon l'une des revendications 1 à 2, **caractérisé par** le fait les points de liaison discrets sont uniformément répartis à l'interface des feuilles.

4. Matériau selon l'une des revendications 1 à 3 , **caractérisé par le fait que** les feuilles (F1) et (F2) sont liées par un adhésif.

5. Matériau selon la revendication précédente, **caractérisé par le fait que** l'adhésif est choisi parmi les adhésifs sensibles à la pression.

6. Matériau selon l'une des revendications 4 ou 5, **caractérisé par le fait que** l'adhésif est choisi parmi les adhésifs fusibles à chaud, dits hot-melts.

7. Matériau selon l'une des revendications 4 ou 5, **caractérisé par le fait que** l'adhésif est un adhésif autoscellable, en particulier le caoutchouc naturel.

8. Matériau selon l'une des revendications 4 à 7, **caractérisé par le fait que** l'adhésif est un adhésif poreux.

9. Matériau selon l'une des revendications 4 à 8, **caractérisé par le fait que** la quantité d'adhésif déposé est comprise entre 1 et 20 g/m², de préférence entre 5 et 10 g/m².

10. Matériau selon l'une des revendication 4 à 9 , **caractérisé par le fait que** l'adhésif est déposé par héliogravure à cylindre tramé.

11. Matériau selon l'une des revendications 1 à 10, **caractérisé par le fait que** l'une des feuilles (F1) est scellable directement ou après enduction d'un produit scellant et que l'autre feuille (F2) a une efficacité en filtration bactérienne BFE supérieure ou égale à celle de la feuille (F1) et que cette efficacité en filtration bactérienne BFE est comprise entre 80 et 100 %.

12. Matériau selon l'une des revendications 1 à 11, **caractérisé par le fait que** la feuille (F1) est une feuille papetière et qu'elle comporte :
- entre 5 et 100 parts en poids de fibres de cellulose, éventuellement modifiée,
- entre 0 et 95 parts en poids de fibres synthétiques, la somme des parts en fibres cellulosiques et synthétiques faisant 100 ;
- entre 0 et 5 % d' un agent de résistance humide, en poids sec de la composition totale de la feuille ,
- entre 0 et 40 % , en poids sec de la composition totale de la feuille ,d'un agent de cohésion.

13. Matériau selon l'une des revendications 1 à 12, **caractérisé par le fait que** la feuille (F2) est une feuille papetière et qu'elle comporte :
- entre 90 et 100 parts en poids de fibres de cellulose, éventuellement modifiée,
- entre 0 et 10 parts en poids de fibres synthétiques, la somme des parts en fibres cellulosiques et synthétiques faisant 100 ;
- entre 0 et 5 % d' un agent de résistance humide, en poids sec de la composition totale de la feuille,
- entre 0 et 40 % , en poids sec de la composition totale de la feuille, d'un agent de cohésion.

14. Matériau selon l'une des revendications 12 ou 13 , **caractérisé par le fait que** les fibres synthétiques sont choisies parmi les fibres d'homopolymères ou de copolymères de polyoléfines, de polyester, de polyamide.

15. Matériau selon l'une des revendications 12 à 14, **caractérisé par le fait que** les fibres synthétiques ont une longueur moyenne comprise entre 1 et 30 mm et un titre moyen compris entre 0,4 et 5 dtex.

16. Matériau selon l'une des revendications 12 ou 13, **caractérisé par le fait que** l'agent de cohésion est choisi parmi les amidons, les alcools polyvinyliques, les polymères acryliques ou acrylates.

17. Matériau selon l'une des revendications 12 à 16, **caractérisé par le fait que** l'agent de cohésion est aussi l'adhésif de liaison entre les feuilles (F1) et (F2) selon les revendications 4 à 8.

18. Matériau selon l'une des revendications 1 à 17 , **caractérisé par le fait que** qu'il est recouvert sur l'une de ses faces d'un adhésif scellant réparti uniformément soit en continu sur toute sa surface soit selon des motifs de grilles ou de zones.

19. Emballage de stérilisation scellable pour les dispositifs médicaux devant être stérilisés, **caractérisé par le fait qu'**il comporte ledit matériau de stérilisation selon l'une des revendications précédentes.

20. Emballage selon la revendication précédente **caractérisé par le fait que** la feuille (F2) ayant la plus grande efficacité en filtration bactérienne BFE est située à l'extérieur de l'emballage.

21. Emballage selon l'une des revendication 19 ou 20, **caractérisé par le fait qu'**il est constitué par ledit matériau de stérilisation selon l'une des revendications 1 à 18 et un film en polymère thermoplastique imperméable aux gaz qui est scellé contre ledit matériau sur une partie de son périmètre au moins.

22. Emballage selon l'une des revendication 19 ou 20, **caractérisé par le fait qu'**il est constitué par ledit matériau de stérilisation selon l'une des revendications 1 à 18 scellé contre lui- même ou une feuille de papier enduite d'un produit scellant comme une couche de polyéthylène extrudé ou du poly (acétate de vinyle).

23. Emballage selon l'une des revendication 19 ou 20, **caractérisé par le fait qu'**il est constitué d'un container rigide et d'un opercule formé par ledit matériau de stérilisation selon l'une des revendications 1 à 18.

## Patentansprüche

1. Siegelbares Sterilisations-Verpackungsmaterial für medizinische Vorrichtungen, die sterilisiert werden müssen, mit einer durchschnittlichen Berstfestigkeit über oder gleich 200 kPa, gemessen gemäß der Norm ISO 2758, einer durchschnittlichen Reißfestigkeit über oder gleich 300 mN, gemessen gemäß der europäischen Norm EN 21974, einer durchschnittlichen Stoßfestigkeit, gemessen gemäß der Norm ASTM D3420 über oder gleich 0,4 J und einer Bakterienfiltrationseffizienz BFE über oder gleich 85%, mit einer flächenbezogenen Masse zwischen 40 und 250 g/m², vorzugsweise zwischen 90 und 250 g/m², gemessen gemäß der Norm ISO 536, das mindestens zwei Sterilisations-Verpackungsfolien (F1) und (F2) umfasst, wobei die eine mindestens direkt oder nach Beschichtung mit einem siegelnden Produkt siegelbar ist, wobei die Folien über eine ihrer Seiten auf nicht reversible Weise miteinander verbunden sind.

2. Material nach Anspruch 1, **dadurch gekennzeichnet, dass** die zwei Sterilisations-Verpackungsfolien (F1) und (F2) durch Verbindungspunkte derart verbunden sind, dass die Verbindungspunkte sich in getrennter Form an der Verbindungsgrenzfläche der Folien befinden.

3. Material nach einem der Ansprüche 1 bis 2, **dadurch gekennzeichnet, dass** die getrennten Verbindungspunkte an der Grenzfläche der Folien gleichmäßig verteilt sind.

4. Material nach einem der Ansprüche 1 bis 3, **dadurch gekennzeichnet, dass** die Folien (F1) und (F2) durch einen Klebstoff verbunden sind.

5. Material nach einem vorstehenden Anspruch, **dadurch gekennzeichnet, dass** der Klebstoff aus den Haftklebstoffen ausgewählt ist.

6. Material nach einem der Ansprüche 4 oder 5, **dadurch gekennzeichnet, dass** der Klebstoff aus den heißschmelzbaren Klebstoffen, genannt Hot-Melts, ausgewählt ist.

7. Material nach einem der Ansprüche 4 oder 5, **dadurch gekennzeichnet, dass** der Klebstoff ein selbstsiegelbarer Klebstoff, insbesondere ein Naturkautschuk, ist.

8. Material nach einem der Ansprüche 4 bis 7, **dadurch gekennzeichnet, dass** der Klebstoff ein poröser Klebstoff ist.

9. Material nach einem der Ansprüche 4 bis 8, **dadurch gekennzeichnet, dass** die aufgebrachte Menge an Klebstoff zwischen 1 und 20 g/m², vorzugsweise zwischen 5 und 10 g/m², beträgt.

10. Material nach einem der Ansprüche 4 bis 9, **dadurch gekennzeichnet, dass** der Klebstoff mittels Rastertiefdruck aufgebracht wird.

11. Material nach einem der Ansprüche 1 bis 10, **dadurch gekennzeichnet, dass** eine der Folien (F1) direkt oder nach Beschichtung mit einem siegelnden Produkt siegelbar ist und dass die andere Folie (F2) eine Bakterienfiltrationseffizienz BFE über oder gleich derjenigen der Folie (F1) besitzt und dass diese Bakterienfiltrationseffizienz BFE zwischen 80 und 100% beträgt.

12. Material nach einem der Ansprüche 1 bis 11, **dadurch gekennzeichnet, dass** die Folie (F1) eine Papierfolie ist und umfasst:
- zwischen 5 und 100 Gewichtsteile gegebenenfalls modifizierte Cellulosefasern,
- zwischen 0 und 95 Gewichtsteile synthetische Fasern, wobei die Summe der Teile der Celluloseund der synthetischen Fasern 100 ausmacht;
- zwischen 0 und 5% eines Feuchtigkeitsbeständigkeitsmittels, bezogen auf das Trockengewicht der Gesamtzusammensetzung der Folie,
- zwischen 0 und 40%, bezogen auf das Trockengewicht der Gesamtzusammensetzung der Folie, eines Zusammenhaltemittels.

13. Material nach einem der Ansprüche 1 bis 12, **dadurch gekennzeichnet, dass** die Folie (F2) eine Papierfolie ist und umfasst:
- zwischen 90 und 100 Gewichtsteile gegebenenfalls modifizierte Cellulosefasern,
- zwischen 0 und 10 Gewichtsteile synthetische Fasern, wobei die Summe der Teile der Celluloseund der synthetischen Fasern 100 ausmacht;
- zwischen 0 und 5% eines Feuchtigkeitsbeständigkeitsmittels, bezogen auf das Trockengewicht der Gesamtzusammensetzung der Folie,
- zwischen 0 und 40%, bezogen auf das Trockengewicht der Gesamtzusammensetzung der Folie, eines Zusammenhaltemittels.

14. Material nach einem der Ansprüche 12 oder 13, **dadurch gekennzeichnet, dass** die synthetischen Fasern aus den Fasern von Homopolymeren oder Copolymeren von Polyolefinen, Polyester, Polyamid ausgewählt sind.

15. Material nach einem der Ansprüche 12 oder 14, **dadurch gekennzeichnet, dass** die synthetischen Fasern eine durchschnittliche Länge zwischen 1 und 30 mm und eine durchschnittliche Feinheit zwischen 0,4 und 5 dtex besitzen.

16. Material nach einem der Ansprüche 12 oder 13, **dadurch gekennzeichnet, dass** das Zusammenhaltemittel aus Amidonen, Polyvinylalkoholen, Acrylpolymeren oder Acrylaten ausgewählt ist.

17. Material nach einem der Ansprüche 12 bis 16, **dadurch gekennzeichnet, dass** das Zusammenhaltemittel auch der Verbindungsklebstoff zwischen den Folien (F1) und (F2) gemäß den Ansprüchen 4 bis 8 ist.

18. Material nach einem der Ansprüche 1 bis 17, **dadurch gekennzeichnet, dass** es auf einer seiner Seiten mit einem siegelnden Klebstoff bedeckt ist, der entweder ununterbrochen über seine gesamte Oberfläche oder in einem Gitter- oder Zonenmuster gleichmäßig verteilt ist.

19. Siegelbare Sterilisationsverpackung für medizinische Vorrichtungen, die sterilisiert werden müssen, **dadurch gekennzeichnet, dass** sie das Sterilisationsmaterial gemäß einem der vorstehenden Ansprüche umfasst.

20. Verpackung gemäß dem vorstehenden Anspruch, **dadurch gekennzeichnet, dass** die Folie (F2), welche die größere Bakterienfiltrationseffizienz BFE aufweist, sich auf der Außenseite der Verpackung befindet.

21. Verpackung gemäß einem der Ansprüche 19 oder 20, **dadurch gekennzeichnet, dass** sie aus dem Sterilisationsmaterial nach einem der Ansprüche 1 bis 18 und einem Film aus einem gasdichten thermoplastischen Polymer besteht, das gegen das Material auf mindestens einem Teil von dessen Umfang gesiegelt ist.

22. Verpackung gemäß einem der Ansprüche 19 oder 20, **dadurch gekennzeichnet, dass** sie aus dem Sterilisationsmaterial nach einem der Ansprüche 1 bis 18 besteht, das gegen sich selbst oder eine Papierfolie gesiegelt ist, die mit einem siegelnden Produkt, wie einer Schicht aus extrudiertem Polyethylen oder Poly(vinylacetat), beschichtet ist.

23. Verpackung gemäß einem der Ansprüche 19 oder 20, **dadurch gekennzeichnet, dass** sie aus einem steifen Behälter und einem Deckel besteht, der durch das Sterilisationsmaterial nach einem der Ansprüche 1 bis 18 gebildet wird.

## Claims

1. Sealable sterilizing packaging material for medical devices that have to be sterilized, having a mean burst strength not less than 200 kPa measured according to the ISO 2758 standard, a mean tear strength not less than 300 mN measured according to the European Standard EN 21974, a mean impact strength not less than 0.4 J measured according to the standard ASTM D 3420 and a bacterial filtering efficiency BFE not less than 85%, having a grammage ranging between 40 and 250 g/m², preferably between 90 and 250 g/m², measured according to the ISO 536 standard and comprising at least two sterilizing packaging sheets (F1) and (F2), at least one of the sheets being sealable directly or after being coated with a sealing product, the said sheets being irreversibly bonded together via one of their sides.

2. Material according to Claim 1, **characterized in that** the two sheets of sterilizing packaging (F1) and (F2) are bonded together by bonding points such that the said bonding points are in a discrete form at the bonding interface of the sheets.

3. Material according to either of Claims 1 and 2, **characterized in that** the discrete bonding points are uniformly distributed at the interface of the sheets.

4. Material according to one of Claims 1 to 3, **characterized in that** the sheets (F1) and (F2) are bonded together by an adhesive.

5. Material according to the preceding claim, **characterized in that** the adhesive is chosen from pressure-sensitive adhesives.

6. Material according to either of Claims 4 and 5, **characterized in that** the adhesive is chosen from hot-melt adhesives, also called hot-melts.

7. Material according to either of Claims 4 and 5, **characterized in that** the adhesive is a self-sealing adhesive, particularly natural rubber.

8. Material according to one of Claims 4 to 7, **characterized in that** the adhesive is a porous adhesive.

9. Material according to one of Claims 4 to 8, **characterized in that** the amount of adhesive deposited ranges between 1 and 20 g/m², preferably between 5 and 10 g/m².

10. Material according to one of Claims 4 to 9, **characterized in that** the adhesive is deposited by halftone gravure printing.

11. Material according to one of Claims 1 to 10, **characterized in that** one of the sheets (F1) is sealable directly or after it has been coated with a sealing product and the other sheet (F2) has a bacterial filtering efficiency BFE not less than that of the sheet (F1) and **in that** this bacterial filtering efficiency BFE ranges between 80 and 100%.

12. Material according to one of Claims 1 to 11, **characterized in that** the sheet (F1) is a paper sheet and **in that** it comprises:
- between 5 and 100 parts by weight of cellulose fibres, the cellulose possibly being modified;
- between 0 and 95 parts by weight of synthetic fibres, the sum of the cellulose fibre parts and the synthetic fibre parts making 100;
- between 0 and 5% of a wet-strength agent by dry weight of the total composition of the sheet;
- between 0 and 40% of a cohesion agent by dry weight of the total composition of the sheet.

13. Material according to one of Claims 1 to 12, **characterized in that** the sheet (F2) is a paper sheet and **in that** it comprises:
- between 90 and 100 parts by weight of cellulose fibres, the cellulose possibly being modified;
- between 0 and 10 parts by weight of synthetic fibres, the sum of the cellulose fibre parts and synthetic fibre parts making 100;
- between 0 and 5% of a wet-strength agent by dry weight of the total composition of the sheet;
- between 0 and 40% of a cohesion agent by dry weight of the total composition of the sheet.

14. Material according to either of Claims 12 and 13, **characterized in that** the synthetic fibres are chosen from fibres homopolymers or copolymers of polyolefins, of polyester, of polyamide.

15. Material according to one of Claims 12 to 14, **characterized in that** the synthetic fibres have a mean length ranging between 1 and 30 mm and a mean linear density ranging between 0.5 and 5 dtex.

16. Material according to either of Claims 12 and 13, **characterized in that** the cohesion agent is chosen from starches, polyvinyl alcohols, acrylic or acrylate polymers.

17. Material according to one of Claims 12 to 16, **characterized in that** the cohesion agent is also the adhesive for bonding between the sheets (F1) and (F2) according to Claims 4 to 8.

18. Material according to one of Claims 1 to 17, **characterized in that** it is covered on one of its sides with a sealing adhesive uniformly distributed either continuously over its entire surface or in patterns of grids or zones.

19. Sealable sterilizing package for medical devices that have to be sterilized, **characterized in that** it comprises the said sterilizing material according to one of the preceding claims.

20. Package according to the preceding claim, **characterized in that** the sheet (F2) having the greater bacterial filtering efficiency BFE is located on the outside of the package.

21. Package according to either of Claims 19 and 20, **characterized in that** it consists of the said sterilizing material according to one of Claims 1 to 18 and a film of gas-impermeable thermoplastic material which is sealed against the said material over at least part of its perimeter.

22. Package according to either of Claims 19 or 20, **characterized in that** it consists of the said sterilizing material according to one of Claims 1 to 18 sealed against itself or a sheet of paper coated with a sealing product such as a layer of extruded polyethylene or of poly(vinyl acetate).

23. Package according to either of Claims 19 or 20, **characterized in that** it consists of a rigid container and of a cover formed by the said sterilizing material according to one of Claims 1 to 18.
